Europäisches Patentamt

European Patent Office

Office européen des brevets

(11) Veröffentlichungsnummer: **0 335 238**
**A1**

## (12) EUROPÄISCHE PATENTANMELDUNG

(21) Anmeldenummer: **89105101.3**

(51) Int. Cl.⁴: **A61M 25/00**

(22) Anmeldetag: **22.03.89**

(30) Priorität: **31.03.88 DE 8804345 U**
**28.05.88 DE 8807003 U**

(43) Veröffentlichungstag der Anmeldung:
**04.10.89 Patentblatt 89/40**

(84) Benannte Vertragsstaaten:
**DE ES FR GB IT SE**

(71) Anmelder: **Fresenius AG**
**Gluckensteinweg 5**
**D-6380 Bad Homburg v.d.H.(DE)**

(72) Erfinder: **Lang, Thomas**
**Haynstrasse 1**
**D-2000 Hamburg 20(DE)**

(74) Vertreter: **Dr. Fuchs, Dr. Luderschmidt**
**Dipl.-Phys. Seids, Dr. Mehler Patentanwälte**
**Sonnenberger Strasse 100**
**D-6200 Wiesbaden(DE)**

(54) **Katheter zur Organperfusion.**

(57) Katheter mit einem Katheterschaft (2) und mit einer seitlichen Öffnung (9) in Abstand von dessem vorderen offenen Ende (4), bei dem in Abstand von der seitlichen Öffnung (9) ein nach hinten im Durchmesser zunehmender kegelförmige Dichtring (7) an der Außenwand des Katheterschaftes (2) vorhanden ist.

## Katheter zur Organperfusion

Die Erfindung betrifft einen Katheter zur Organperfusion mit einem Katheterschaft, der eine seitliche Öffnung im Abstand von dessen distalem Ende sowie eine atraumatische Aufweitung und das proximale Ende Anschlußmittel für medizinische Geräte aufweist.

Die Perfusion dient zur Konservierung von menschlichen Spenderorganen, bei denen die Organe mit dem Perfusat durchspült werden. Zu diesem Zweck weist das Perfusionssystem einen geeigneten Perfusionskatheter auf, der in ein Organ des menschlichen Körpers eingeführt wird. Dieser Katheter besteht aus einem aus Kunststoff gefertigten Schlauch, dessen Ansatzstück sich am proximalen Ende trichterförmig vergrößert und damit eine Adaptierung an das Perfusionssystem möglich macht, so daß eine übergangslose Verbindung ohne Querschnittsänderung erfolgt. An diese trichterförmige Vergrößerung können medizinische Geräte, wie Spritzen, Kanülen, Schlauchleitungen o.dgl. angeschlossen werden, die ebenfalls einen trichterförmigen bzw. konischen Anschluß aufweisen. In der Regel verfügen die Perfusionskatheter über eine atraumatisch geformte Spitze an ihrem distalen Ende, um beim Einführen des Katheters in das Organ bzw. in das Organgefäß eventuelle Gefäßschädigungen zu verhindern. Nach dem Anschlingen des Katheters und nach Inzision des Organgefäßes wird der Katheter in das Organgefäß eingeführt. Nach der Plazierung des Katheters erfolgt die Fixation des Katheters mittels Ligatur. Diese Fixation brachte jedoch den Nachteil mit sich, daß die Inzisionsstelle nicht ausreichend abgedichtet wurde und dadurch das Perfusat aus der Inzisionsstelle austrat. Außerdem wurde der Perfusionskatheter nicht ausreichend an der Inzisionsstelle arretiert.

Die Aufgabe der Erfindung besteht nun darin, einen Katheter zur Organperfusion derart auszubilden, daß dieser die Inzisionsstelle des zu perfundierenden Organs abdichtet, sich selbst lagefixiert und einen ausreichenden Abfluß gewährleistet.

Erfindungsgemäß wird die Aufgabe dadurch gelöst, daß der Katheterschaft an seiner Außenwand im Abstand von seiner seitlichen Öffnung wenigstens einen sich in Richtung des proximalen Endes konisch erweiternden Dichtring aufweist. Der Katheterschaft kann aber auch mehrere, zueinander beabstandete Dichtringe aufweisen. Diese können sowohl integrierter Bestandteil des Katheterschaftes als auch nachträglich auf den Katheterschaft aufgebrachte Teile sein.

Der Katheter, der aus medizinisch verträglichem Kunststoff, beispielsweise Polyurethan besteht, wird durch die Inzisionsstelle in das Organgefäß eingeführt, wobei der bzw. die Dichtringe mit ihrem verjüngten Ende zuvorderst in das Organ eindringen. Bei weiterem Vorschieben des Katheters wird die Inzisionsstelle durch die konische Form der Dichtringe langsam erweitert bis sich das Gefäß hinter dem breiten Ende des konischen Dichtringes zusammenzieht und um den Katheterschaft legt. Zusätzlich kann der Katheter distal des Dichtringes mittels Ligatur fixiert werden. Durch den konusartigen Dichtring erfolgt eine vollständige Abdichtung der Inzisionsstelle und eine Arretierung des Perfusionskatheters im Organgefäß.

Um das Einführen des Perfusionskatheters zu erleichtern, können die konusartigen Dichtringe aus einem weicheren Kunststoffmaterial als der Katheterschaft bestehen, damit sich der Dichtring beim Einführen des Katheterschaftes beim passieren der Inzisionsstelle zusammenziehen kann und dadurch die Inzisionsstelle nicht unnötig erweitert bzw. gedehnt wird.

In einer besonderen Ausbildungsform der Neuerung können die konusartigen Dichtringe aus dem gleichen Material wie der Katheterschlauch bestehen und mit diesem integriert sein. Dies bringt erhebliche, fertigungstechnische Vorteile mit sich. In einer anderen Ausführungsform können die Dichtringe nachträglich auf den Katheterschaft, beispielsweise durch Aufschrumpfen, aufgebracht sein.

Die seitliche Öffnung neben der offenen Spitze ergibt ein großes Lumen, um einen ausreichenden Abfluß zu gewährleisten, so daß die zu perfundierenden Organe in kurzer Zeit mit den hierfür verwendeten Lösungen, beispielsweise Eurocollins-Lösungen, perfundiert werden können. Vorzugsweise beträgt der Abstand der seitlichen Öffnung im Katheterschaft mit einem Außendurchmesser von 6-10 mm von der unter einem Winkel von 30-60° abgewinkelten offenen Spitze 3-6 mm und die seitliche Öffnung ist in Längstrichtung des Katheterschaftes 6-8 mm lang und in Umfangsrichtung 3-5 mm breit. Ganz besonders bevorzugt ist eine um 45° abgewinkelte Spitze des Katheters, bei dem das vordere Ende der seitlichen Öffnung mit dem abgeschrägten Ende der Katheterspitze fluchtete und die seitliche Öffnung einen mit der Länge der Abschrägung übereinstimmenden Abstand von der äußersten Katheterspitze aufweist. Die Öffnung ist 7 mm lang und 4 mm breit bei einem Außendurchmesser des Katheters von 6 oder 8 mm.

Der Abstand des vorderen, an der Außenoberfläche des Katheterschaftes befestigten Endes des Dichtringes vom hinteren Ende der seitlichen Öffnung beträgt 7-10 mm, wobei der Dichtring eine Länge von 5-12 mm aufweis. Vorzugsweise beträgt der Abstand distal von der Katheterspitze etwa 15 mm.

Der Außendurchmesser des Dichtringes am freien hinteren Ende kann 8-12 mm betragen, vorzugsweise 9,9-11,3 mm.

Der Katheter kann eine Länge von 30 cm bis 1 m aufweisen und der Katheterschaft ist am hinteren Ende zum Anschluß an entsprechende Apparaturen im Durchmesser leicht trichterförmig aufgeweitet, so daß ein Aufschieben auf einen Schlauchanschlußstutzen möglich ist oder ist mit einem sogenannten Lueranschluß versehen, der so dimensioniert ist, daß die Querschnittsverengung im Bereich des Lueranschlßes im Vergleich zum Innendurchmesser des Katheters minimal ist. Dies gewährleistet einen ausreichenden Abschluß, so daß mit dem erfindungsgemäßen Katheter Organe in sehr kurzer Zeit perfundiert werden können.

An einem Ausführungsbeispiel soll die Neuerung näher erläutert werden.

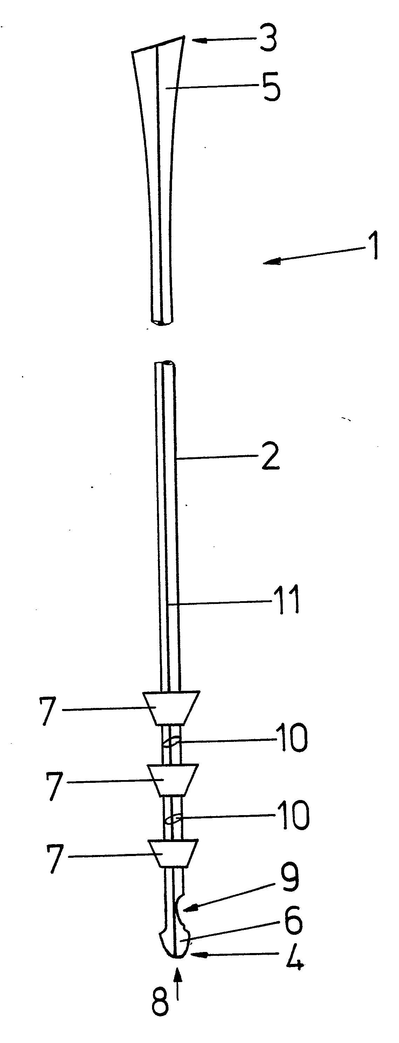

Die beiliegende Zeichnung zeigt einen Perfusionskatheter 1, der aus einem Katheterschaft 2 besteht. Dieser weist ein proximales Ende 3 und ein distales Ende 4 auf. Das proximale Ende 3 ist dabei trichterförmig ausgebildet und dient als Anschlußmittel 5 für medizinische Geräte wie Spritzen, Kanülen, Schlauchleitungen usw., die einen konusförmigen Anschluß besitzen. Deren Konus wird in das trichterförmige Anschlußmittel 5 eingeführt und durch die federelastische Eigenschaft des aus Siliconmaterial bestehenden Katheterschaftes 2 festgeklemmt, so daß eine übergangslose Verbindung ohne Querschnittsänderung vom Perfusionssystem zum Perfusionskatheter 1 erfolgt. Über diesen trichterförmigen Anschluß 5 wird später das Perfusat in den Katheterschaft 2 und weiter in das Organgefäß geleitet, um dieses zu durchspülen.

Das distale Ende 4 des Katheterschaftes 2 weist eine atraumatisch geformte Spitze 6 auf, die vorzugsweise die Form einer Abacus-Wulst besitzt. Dadurch werden Gefäßschädigungen beim Einführen des Perfusionskatheters 1 verhindert.

Im Bereich des distalen Endes 4 des Katheterschaftes 2 sind ein oder mehrere sich in Richtung des proximalen Endes 3 konisch erweiternde Dichtringe 7 beabstandet zueinander angeordnet. Diese können integrierter Bestandteil des Katheterschaftes 2 sein und aus dem gleichen Material wie der Katheterschaft 2 bestehen. Andererseits können diese Dichtringe 7 auch aus einem anderen, vorzugsweise weicheren Material bestehen und nachträglich auf den Katheterschaft 2, beispielsweise durch Aufschrumpfen, aufgebracht sein.

Das distale Ende 4 des Katheterschaftes 2 weist das Hauptauslaßlumen 8 auf. In unmittelbarer Nachbarschaft dieses Hauptauslaßlumens 8 ist eine zusätzliche seitliche Öffnung 9 angebracht, die bei eventuellem Anliegen des Hauptauslaßlumens 8 an der Gefäßintima eine Perfusion sicherstellt.

In einer weiteren Ausbildung der Neuerung, insbesondere wenn mehrere konusartige Dichtringe 7 auf dem Katheterschaft 2 beabstandet zueinander angeordnet sind, können zwischen diesen Dichtringen spezielle vormarkierte Trennstellen 10, die als Sollbruchstellen ausgebildet sein können, angeordnet sein. Dadurch läßt sich der Perfusionskatheter 1 an das spezifische Organ anpassen und die Einführtiefe bestimmt werden, in dem der Katheterschaft 2 an einer bestimmten Trennstelle 10 abgeschnitten wird, so daß der Katheter 1 gekürzt und dementsprechend organspezifisch angepasst wird. Außerdem kann der Perfusionskatheter in unterschiedlichen Durchmessern für jeweils spezifische Organe ausgebildet sein. Weiterhin kann der Katheterschaft 2 mit einem Röntgenstreifen 11 versehen sein. Der Perfusionskatheter 1 wird mit seiner am distalen Ende 4 angebrachten atraumatisch geformten Spitze 6 zuvorderst durch die Inzisionsstelle in das Organgefäß eingeführt. Dabei passiert der Dichtring 7 die Inzisionsstelle und erweitert diese durch seine konische Form so weit, bis die Inzisionsstelle den größten Durchmesser des konusartigen Dichtringes 7 passiert hat und sich hinter diesen um den Katheterschaft 2 legt. Dadurch wird die Inzisionsstelle des Organgefäßes vollständig und sicher abgedichtet und der Perfusionskatheter 1 im Organ lagefixiert.

## Ansprüche

1. Katheter zur Organperfusion mit einem Katheterschaft (2), der eine seitliche Öffnung (9) im Abstand von dessen distalem Ende (4) sowie eine atraumatische Aufweitung (6) und am proximale Ende (3) Anschlußmittel (5) für medizinische Geräte aufweist, dadurch gekennzeichnet, daß der Katheterschaft (2) an seiner Außenwand im Abstand von seiner seitlichen Öffnung (9) wenigstens einen sich in Richtung des proximalen Endes (3) konisch erweiternden Dichtring (7) aufweist.

2. Katheter nach Anspruch 1, dadurch gekennzeichnet, daß der Katheterschaft (2) mehrere zueinander beabstandete Dichtringe (7) aufweist.

3. Katheter nach den Ansprüchen 1 und 2, dadurch gekennzeichnet, daß die Dichtringe (7) integrierter Bestandteil des Katheterschaftes (2) sind.

4. Katheter nach den Ansprüchen 1 und 2, dadurch gekennzeichnet, daß die Dichtringe (7) nachträglich auf den Katheterschaft (2) aufgebrachte Teile sind.

5. Katheter nach Anspruch 1, dadurch gekennzeichnet, daß dieser aus medizinisch verträglichem Kunststoff, beispielsweise Polyurethan, besteht.

6. Katheter nach den Ansprüchen 1 bis 5, dadurch gekennzeichnet, daß die Dichtringe (7) aus dem gleichen Material wie der Katheterschaft (2) bestehen.

7. Katheter nach den Ansprüchen 1, 2 und 4, dadurch gekennzeichnet, daß die Dichtringe (7) aus einem Kunststoff bestehen, der weicher ist als das Material des Katheterschaftes (2).

8. Katheter nach Anspruch 1, dadurch gekennzeichnet, daß der Abstand der seitlichen Öffnung (9) im Katheterschaft (2) mit einem Außendurchmesser von 6-10 mm von der unter einem Windel von 30-60° abgewinkelten offenen Spitze 3-6 mm beträgt und die Öffnung in Längsrichtung des Katheterschaftes (2) 6-8 mm lang und in Umfangsrichtung 3-5 mm breit ist.

9. Katheter nach Ansprüchen 1-4, dadurch gekennzeichnet, daß der Abstand des vorderen, an der Außenoberfläche des Katheterschaftes (2) befindlichen Endes des Dichtringes (7) vom hinteren Ende der seitlichen Öffnung (9) 7-10 mm beträgt und der Dichtring (7) eine Länge von 5-12 mm aufweist.

10. Katheter nach Ansprüchen 8 und 9, dadurch gekennzeichnet, daß der Außendurchmesser des Dichtringes (7) am hinteren freien Ende 8-12 mm beträgt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-2 357 308 (ASTRA-SJUCO AB) <br> * Figur 1; Seite 4, Zeilen 3-20 * <br> --- | 1-10 | A 61 M 25/00 |
| X | US-A-3 626 950 (SCHULTE) <br> * Spalte 1, Zeile 70 - Spalte 2, Zeile 26; Figur 5 * <br> --- | 1-10 | |
| X | US-A-3 903 895 (ALLEY et al.) <br> * Figuren 1,2; Spalte 3, Zeilen 3-21 * | 1 | |
| A | <br> --- | 2-10 | |
| A | EP-A-0 185 865 (INTERMEDICAT GmbH) <br> * Anspruch 1; Figur 1 * <br> ----- | 1-7 | |

RECHERCHIERTE
SACHGEBIETE (Int. Cl.4)

A 61 M

Der vorliegende Recherchenbericht wurde für alle Patentansprüche erstellt

| Recherchenort | Abschlußdatum der Recherche | Prüfer |
|---|---|---|
| DEN HAAG | 03-07-1989 | RAKOWICZ,J.M. |